# EUROPEAN PATENT SPECIFICATION

(11) **EP 4 551 070 B1**
(45) Date of publication and mention of the grant of the patent: **09.12.2026**
(21) Application number: 23744863.4
(22) Date of filing: 10.07.2023
(51) Int. Cl.: A45D 34/04

(54) **DEVICE FOR DISPENSING AND APPLYING LOTION**
VORRICHTUNG ZUM ABGEBEN UND AUFTRAGEN VON LOTION
DISPOSITIF DE DISTRIBUTION ET D'APPLICATION DE LOTION

(30) Priority: 08.07.2022 GB 202210055
(43) Date of publication of application: 14.05.2025
(73) Proprietor: Solex Sun Protection Limited, Westcliff-On-Sea Essex SS0 9PE (GB)
(72) Inventor: TOWNSEND, Peter John, Rayleigh Essex SS6 9LD (GB)
(74) Representative: Hindles Limited
(86) International application number: PCT/GB2023/051814
(87) International publication number: WO 2024/009114

(56) References cited:
- JP-B2- 6 155 082
- US-A- 4 747 720
- US-A- 6 129 469
- US-A1- 2001 031 171
- US-A1- 2013 108 350
- US-A1- 2020 047 958
- US-A1- 2020 205 552
- US-B2- 11 104 488

## Description

### Field of the invention

The present specification relates to a device for dispensing and applying a lotion, typically a sun-cream applicator.

### Background to the invention

People who spend significant amounts of time outdoors are particularly susceptible to the damaging effect the Sun's radiation can have on their skin. It is well recognised that lotions such as sun-cream can provide valuable protection in this regard. However, the process of applying sun-cream often results in cream getting distributed to areas where it is not required, such as the palms of the hands, where it can also result in problems due to the hands becoming slippery for example. This can be a particular problem for sports people, such as golfers, who rely on having a good grip of their equipment to enjoy and succeed in their sport. An example of a device for dispensing and applying a lotion is known from JP 6 155082 B2.

It is in this context that the present disclosure has been devised.

### Summary of the invention

The present invention relates to a device for dispensing and applying lotion as defined by claim 1, the device comprising: a body, the body being resiliently deformable and defining a receptacle for storing lotion therein; a head having defined therein one or more apertures extending over an area of a rigid first surface of the head, wherein the body and the head are connected such that a fluid connection exists between the receptacle and the one or more apertures of the head; and an absorbent applicator capping the rigid first surface of said head.

Thus, lotion can be applied by first squeezing the body to transfer a portion of lotion stored in the receptacle into the head, and through the one or more apertures to load the absorbent applicator with the lotion (e.g. sun-cream). Once loaded with lotion, the absorbent applicator can be brought into contact with the skin of the user. In this way, the lotion is transferred from the device, specifically from the absorbent applicator of the device, onto the skin of the user as required. The absorbent applicator sits snuggly on the head portion and caps the head of the device. The device enables lotion such as sun-cream to be carried around by the user and accessed by the user as and when required. Lotion such as sun-cream which is stored in the receptacle of the device may be applied directly to the skin of the user via the absorbent applicator, thereby minimising unnecessary spread of lotion to unwanted areas. Importantly, the rigid first surface of the head ensures that the absorbent applicator does not significantly deform when rubbed over the skin, allowing the lotion not just to be applied to the skin, but also to be rubbed into the contours of the skin, as is important for some lotions, such as sun-cream in order to provide adequate and consistent coverage.

The body is typically sized so that it may be comfortably gripped in an adult hand. Thus, the device may be comfortably gripped in an adult hand.

The body typically is typically elongate in shape, with a longitudinal axis which runs from a head-end at the end of the body which connects to the head, to an opposite bottom end. The length of the body from the head end to the opposite bottom end may be greater than 30 mm, such as greater than 50mm, or even greater than 70mm. The length of the body from the head end to the opposite bottom end may be less than 150 mm, such as less than 130 mm, or even less than 110 mm. The length of the body from the head end to the opposite bottom end may be around 90 mm. The body may have a minimum breadth of greater than 30 mm, such as greater than 40 mm, or even greater than 50 mm. The body may have a maximum breadth of less than 90 mm, such as less than 80 mm, or even less than 70 mm. The breadth of the body may be around 60 mm. The body may have a maximum depth of less than 56 mm, such as less than 51 mm, or even less than 44 mm. The body may have a minimum depth of greater than 20 mm, such as greater than 25 mm, or even greater than 32 mm. The breadth of the body may be around 38 mm.

Thus, the device is of a size which can be carried and used with ease by most people.

It will be understood that the body being resiliently deformable means that the body may be compressible under force, and thereafter upon release of the force returning to its original uncompressed form. The force used to compress the body may be applied by the hand of a user, for example by squeezing the body within the palm or with the fingers.

The receptacle may contain a volume of 500 ml or less, or less than 500 ml, or less than 400 ml, or less than 300 ml, or less than 250 ml, or less than 200 ml, or less than 100 ml, or 75 ml, or less than 75 ml. The receptacle may contain a volume of 50 ml, or more than 50 ml, or more than 75 ml, or more than 100 ml, or more than 200 ml, or more than 250 ml.

Each of the apertures may be of circular cross-section, although other cross-sectional shapes, such as square, triangular, oval, elliptic, polygonal, are possible. The cross-sectional area of an aperture may be less than 12 mm², possibly less than 6 mm², or even less than 3 mm². The cross-sectional area of an aperture may be greater than 0.1 mm², possibly greater than 0.4 mm², or even greater than 0.8 mm². Thus, lotion can easily pass through each aperture, but there is still sufficient material forming the rigid first surface of the head that the absorbent applicator is adequately supported thereon.

A total cross-sectional area of the one or more apertures may be greater than 10 mm². The total cross-sectional area of the one or more apertures may be less than 5 cm². Thus, the one or more apertures are sized so as to permit a useful quantity of lotion therethrough to load the absorbent applicator.

Each of the apertures may have a depth of 5 mm or less than 5 mm, or less than 4 mm, or less than 3 mm, or less than 2 mm, or less than 1.5 mm. Each of the apertures may have a depth of greater than 0.5 mm. The depth of an aperture may be the direct distance from an edge of the aperture on the rigid first surface of the head to an edge of the aperture on an opposing second surface of the head, opposite the first surface.

The fluid connection between the receptacle and the one or more apertures may be a valved fluid connection. In other words, the fluid connection may not always be open. Whether the fluid connection is open or not may depend on the state of a valve. In other words, it will be understood that a fluid connection is still provided between the receptacle of the body and the one or more apertures of the head even when the receptacle and the one or more apertures are temporarily prevented from being in fluid communication by a valve, as long as it is possible to bring the receptacle into fluid communication with the one or more apertures using the valve.

The absorbent applicator may be made of a foamed material, which may be referred to as foam. The absorbent applicator may be porous. The absorbent applicator may comprise a layer made of foam and which is formed to conform to the first surface of the head portion. In this way the absorbent applicator caps the first portion of the head portion, by sitting snuggly on the first portion of the head portion. The absorbent applicator may be made of polyester, for example polyester fur.

The absorbent applicator may comprise a layer. The absorbent applicator may comprise a foam layer. The absorbent applicator has a thickness. When the absorbent applicator comprises a foam layer, the thickness of the absorbent applicator is the thickness of the foam layer from which it is comprised. This thickness of the absorbent applicator may be greater than 0.5 mm, such as greater than 1 mm, or even greater than 1.5 mm. The thickness of the absorbent applicator may be less than 10 mm, such as less than 8 mm, or even less than 6 mm. The absorbent applicator may be around 1.5 mm thick. An absorbent applicator with a thickness in the indicated range is sufficiently thick to absorb a quantity of lotion, such as sun-cream, which may be usefully applied to a user, but will not absorb an excess of lotion which, quite apart from being uneconomical, could otherwise cause the user's hands to become slippery.

The head of the device may define a cavity which contributes to the fluid connection between the receptacle and the one or more apertures. That is to say, the head of the device may be hollow, thereby forming a cavity within the device, which cavity may form part of the fluid connection between the receptacle and the one or more apertures

The cavity in the head of the device may have a cross-sectional area, which with increasing distance from the body decreases in area, before reaching a minimum area and then increasing in area again. The cross-sectional shape of the cavity may vary with increasing distance from the body.

The rigid first surface of the head of the device has a form to facilitate the application of lotion from the absorbent applicator onto a person, in particular onto the face of a person.

At least a region of the first surface of the head defines a substantially concave profile in a first direction. Thus, the shape of the first surface can facilitate the effective application of lotion to bumps and protruding areas of the head or body. At least a region of the first surface of the head defines a substantially convex profile in a second direction. Thus, the shape of first surface can additionally facilitate the effective application of lotion to creases, dips and depressions in the head and body. The second direction may be transverse to the first direction. Thus, a head shape comprising both convex and concave profiles can be used to apply lotion to substantially any part of the head and body.

The absorbent applicator caps the head of the device. The absorbent applicator typically forms an absorbent layer whose form matches that of the head of the device. The form of the applicator surface is determined by the surface form or shape of the head. The surface form or shape of the head, being the outer surface form or shape, is such that contact between the absorbent applicator and the skin of the user is enhanced. In this way lotion may be applied to the skin of a user, for example to the skin of the face of a user in a manner which optimises an even and complete distribution of lotion.

The rigid first surface of the head may be convex shaped. The rigid first surface of the head may comprise a peak. The rigid first surface of the head may comprise a plurality of peaks, for example a first peak and a second peak. The first peak and the second peak may together form a saddle shaped first surface of the head. The first peak and the second peak may be substantially equal in size. The first peak may extend to a height greater than a height of the second peak.

The rigid first surface of the head may comprise a central area with a concave cross-section in a first plane, and a convex cross-section in a second orthogonal plane. In the first plane, a convex cross-section may be present at one or both sides of the concave cross-section.

The rigid first surface of the head may comprise a plurality of peaks, typically two peaks. The rigid first surface of the head may comprise two peaks with a connecting ridge.

The rigid first surface may define a saddle shape.

The one or more apertures of the device may be a plurality of apertures.

Apertures may be located at or around the apex of one or more of the peaks of the head. Apertures may be located in a concentric arrangement around the apex of one or more of the peaks of the head. One or more apertures may be located along the connecting ridge of the head. Apertures may be located at or around the centre point of the connecting ridge of the head. Apertures may be located in a concentric arrangement around the centre point of the connecting ridge of the head.

It may be that the one or more apertures are distributed throughout the rigid first surface of the head.

The one or more apertures may comprise a single aperture, or two or more apertures, such as more than 4 apertures, or more than 12 apertures, or more than 24 apertures, or more than 48 apertures, or more than 96 apertures. The number of apertures may be less than 400 apertures, possibly less than 350 apertures, or less than 300 apertures, or less than 200 apertures or less than 150 apertures. The number of apertures may be for example 106 apertures.

The device may further comprise a lotion valve between the body and the head, wherein said lotion valve has a first configuration, in which first configuration passage of lotion from the body to the one or more apertures via the fluid connection is restricted or prevented, and a second configuration, in which second configuration lotion may pass from the body to the one or more apertures via the fluid connection.

The head of the device may be connected to the body by way of a rotatable mount, so that the head may rotate (or be rotated) relative to the body around a longitudinal axis of the device.

The head of the device may be connected to the body by way of a screw attachment, or a screw mount, typically operating around a longitudinal axis of the device.

The head may be rotatable around a longitudinal axis of the device between a first position relative to the body and a second position relative to the body. It may be that rotation of the head relative to the body is configured to cause a change in the configuration of the lotion valve. In the first position the lotion valve may be in its first configuration. In the second position the valve may be in its second configuration. The angle between the first and second positions may be 180° or less, or 10° or greater. The angle may for example be around 90°.

The head and the body may each have a cross-section which match with each other in the first position, but do not match with each other in the second position when the head is rotated relative to the body. For example, the head may have an oval cross-section and the body may have an oval cross-section. In the first position, the long axis of the oval cross-section of the head may be parallel to the long axis of the oval cross-section of the body. In the second position, the long axis of the oval cross-section of the head may be perpendicular to the long axis of the oval cross-section of the body. In this way, by comparing the position of the long axis of the oval cross-section of the head to the long axis of the oval cross-section of the body a user of the device can easily determine whether the lotion valve is in its second (open) configuration, or in its first (restricted or closed) configuration, for example by touch alone. Thus, the device is suitable for use even in busy situations where the user may not have the ability or desire to look at the device to determine whether the lotion valve is in the closed (first) configuration or the open (second) configuration.

The absorbent applicator may be formed from a flexible material. The absorbent applicator may be porous.

If the device comprises an absorbent applicator which is formed from a flexible material, the applicator will tend to adapt to smaller contours of the skin of the user when applying lotion, thereby enhancing the even application of lotion such as suncream to the benefit of the intended skin protection.

If the device comprises an absorbent applicator which is porous, this enhances the ability of the absorbent applicator to be loaded with lotion prior to application.

The device may comprise engagement means which serves to retain in place the absorbent applicator capping the rigid first surface of said head. It may be that the engagement means act as locators when assembling the absorbent applicator onto the rigid first surface of the head.

The head may comprise the engagement means, or the engagement means may be present on the head. For example, the engagement means may comprise a protrusion or protrusions on the head around which the absorbent applicator forms. The protrusion or protrusions may indent into, or otherwise engage with for example by passing through a securing hole (or holes) of, an absorbent applicator, and thereby help to retain the absorbent applicator in place.

The absorbent applicator may comprise the engagement means, or the engagement means may be present as part of the absorbent applicator. For example, the engagement means may comprise an adhesive.

The engagement means may be comprised of a first element being part of the head, and a second element being part of the absorbent applicator, which first and second element interact in an interrelated manner. For example, a first element of engagement means may comprise protrusions protruding from the head and the second element may comprise indentations in the absorbent applicator which are configured to receive the protrusions of the head.

The engagement means may comprise a clamp. The clamp may be or may comprise one or more clamp elements. The clamp may clamp to or around the head, for example around a circumference of the head, sandwiching a rim of the absorbent applicator between the ring clamp and the head.

The clamp may comprise a set of first cooperating elements. The head may comprise a set of second cooperating elements. Each element of the set of second cooperating elements typically has a partner element in the set of first cooperating elements. Each element of the set of second cooperating elements typically has a partner element in the set of first cooperating elements with which it forms a pair of cooperating elements. When the clamp is clamped to or around the head, each first cooperating element may engage with a corresponding second cooperating element. When the absorbent applicator is mounted on the head and the clamp is mounted to or around the head, each first cooperating element may engage with a corresponding second cooperating element in such a way that the pair of engaging cooperating elements passes through a securing hole of the absorbent applicator. Thus, by cooperating elements passing through the securing hole of the absorbent applicator, the absorbent applicator is even more securely retained in place on the head. By being securely retained at multiple points, even with some slight degradation of the absorbent applicator over time, it will still remain securely attached to the head and therefore allow the device to continue being used.

The set of first cooperating elements may comprise at least one cooperating element, such as a plurality of cooperating elements. The set of first cooperating elements may comprise fewer than ten cooperating elements, such as five or fewer cooperating elements. Each cooperating element of the set of first cooperating elements may have substantially the same shape and size as each other cooperating element of the set of first cooperating elements.

The set of second cooperating elements may comprise at least one cooperating element, such as a plurality of cooperating elements. The set of second cooperating elements may comprise fewer than ten cooperating elements, such as five or fewer cooperating elements. Each cooperating element of the set of second cooperating elements may have substantially the same shape and size as each other cooperating element of the set of second cooperating elements.

A one of the set of first cooperating elements and the set of second cooperating elements may each define an opening therein, for receiving a corresponding securing protrusion of each of the other of the set of first cooperating elements and the set of second cooperating elements. The opening may be defined in each of the set of second cooperating elements, and the securing protrusion may be provided at each of the set of first cooperating elements.

The engagement means may be separately identifiable from the head or the absorbent applicator, such as an adhesive attachment contributing to the attachment of the rigid first surface of the head to the absorbent applicator.

The device may additionally comprise an air inlet allowing air to enter the receptacle.

The air inlet typically comprises a stop valve, thereby allowing air from the surrounding atmosphere to enter the receptacle, but preventing or resisting air, or any other fluid within the receptacle, to escape. When the body is deformed, for example by squeezing, pressure is built up within the receptacle. If a stop valve prevents or resists air escaping through the air inlet, the main pressure release mechanism may then be through the fluid connection to the one or more apertures. Pressure release may occur by the movement of lotion through the one or more apertures via the fluid connection.

The device may be arranged such that air enters the receptacle when the body, having been resiliently deformed so as to compress the receptacle, is permitted to return to an original shape increasing the volume of the receptacle.

The presence of an air inlet to enable the body to return to its original shape allows the body to be re-compressed in order to again load the absorbent applicator for example.

The air inlet may be arranged to cause air to be directed to a distal end of the receptacle, away from a proximal end of the receptacle, the proximal end being closer to the head than the distal end.

The air inlet may be arranged to cause air to enter the receptacle at the distal end, away from the proximal end, the proximal end being closer to the head than the distal end.

The air inlet may comprise an air inlet entry, through which air typically enters the device. The air inlet may comprise an air inlet passage, through which air which has entered the device passes. The air inlet passage may be formed within the material forming the body of the device. The air inlet passage may comprise a tube which is configured to extend to a position in the receptacle. The air inlet passage may comprise a tube which is configured to extend to the distal end of the receptacle.

The air inlet entry may be located at the bottom, or distal, end of the body of the device.

The air inlet entry may be located in the head of the device. The air inlet entry may be located in a surface of the head of the device. For example, the air inlet entry may be in a surface of the head of the device which surface is hidden from view when the cross-section of the head and the cross-section of the body match with each other in the first position.

The device may comprise carrying means with which the device may be carried. The carrying means may be a handle or a strap.

The carrying means may be attached to the rest of the device at the bottom or distal end of the body. The bottom or distal end of the body, i.e. at the end of the body opposite to the head end of the body) may include a through-hole, through which the carrying means may pass.

If the device is carried by suspending via carrying means at the bottom or distal end of the body, the device will hang in a way such that the head end is lower than the bottom end. In this way, when the device is suspended by the through-hole, lotion present in the receptacle of the body will tend to accumulate away from the distal or bottom end of the body.

The air inlet entry may be located within the through-hole at the bottom end of the body of the device.

If a lotion valve is present and in a closed (second) configuration, when the device is suspended by the through-hole, lotion will tend to accumulate in a region of the receptacle towards the closed lotion valve. If the lotion valve is then opened, lotion may move into the cavity of the head.

If the lotion valve is in a restricted (first) configuration, or if no lotion valve is present in the device, when the device is oriented so that the bottom end of the body is positioned higher than the head end, lotion may accumulate in the cavity of the head. The device typically is typically oriented in this way when it is suspended by the through-hole. The viscosity of the lotion and/or the surface tension of the lotion, in combination with the physical characteristics of each of the one or more apertures, such as the dimensions of the one or more apertures and possibly the nature of the material of the head, may ensure that under static conditions any lotion which has accumulated in the cavity of the head under the force of gravity, remains there and does not seep out of the one or more apertures.

The device may additionally comprise a cap which is configured to be removably retained on the head, the cap thereby when mounted covering the adsorbent applicator and protecting it from the surrounding atmosphere.

The cap may fit onto the head via a friction fit.

The cap may comprise a sealing element or a plurality of sealing elements. The sealing element or elements may extend from an internal surface of the cap. The sealing element or elements may extend from an internal surface of the cap. The sealing element or elements may extend in a direction parallel to a central axis of the receptacle. The sealing element or elements may extend in such a way that they engage with, for example by entering into and thereby blocking, an aperture or apertures in the head when the cap is mounted on the device. In this way the sealing element or elements may seal the receptacle when the cap is mounted to prevent suncream from drying out. This is particularly advantageous if the device is not utilised for an extended period of time. The sealing element(s) may be in the form of a protrusion shaped to enter and fill the aperture or apertures in the head, when the cap is mounted on the device.

The cap may provide a stand on which the device may be stood.

The cap may be such that the device may be stood on the cap upon a supporting surface, such as a table top. The cap may be such that the device may be stood on the cap upon a supporting surface, in an orientation whereby the distal end of the body is maintained uppermost. The cap may comprise a ridge on which the device may be supportable on a supporting surface. The ridge may surround a flat surface of the cap.

The device may comprise a mirrored surface.

The mirrored surface may be a flat mirrored surface. The mirrored surface may be detachable. In this way the mirror may be utilised by a user when using the device to apply sun-cream to his or her face, to aid the application of the sun-cream. The mirror may be part of the cap. The cap may comprise a flat surface which is a mirror or which comprises a mirrored surface. The cap may comprise a flat surface which is a mirror or which comprises a mirrored surface, and a ridge surrounding the flat surface, upon which ridge the cap and, when device and cap are attached, the device may be stood or supported.

The device may comprise a switch. The switch may be in the form of a pressure sensor. The pressure sensor may be configured to detect when the absorbent applicator is pressed against the skin of a user during the act of utilising the applicator device to apply lotion such as sun-cream to the skin. The pressure sensor may only function when it is activated. In other examples, switch may be activated by rotating the head of the device relative to the body by a predetermined angle, such as 180°. The switch may not be activated unless the head of the device is rotated relative to the body by the predetermined angle, such as 180°. It may be that the switch is deactivated by rotating the head of the device relative to the body in an opposite direction.

The switch may be functionally connected to an indicator, such as a light emitter and/or a sound emitter. In this way, when the switch, such as the pressure sensor, is activated, light may be emitted by the light emitter, and/or, as appropriate, sound may be emitted by the sound emitter. It will be understood that where the switch is a pressure sensor, the emission of sound and/or light may be caused by detection of contact between the absorbent applicator and the skin of the user.

The emission of sound and/or light can cause a distraction, which can make it easier to apply lotions to small children, who might otherwise move around too much.

### Description of the Drawings

Examples of the present disclosure will now be illustrated with reference to the following Figures in which:
Figure 1 shows an exploded diagram of a device for dispensing and applying lotion in accordance with an aspect of the present disclosure.
Figure 2(a) shows a cross-sectional view of a device for dispensing and applying lotion in accordance with an aspect of the present disclosure in exploded form; Figure 2(b) shows the same cross-sectional view in unexploded form.
Figure 3 shows an external view of a device for dispensing and applying lotion in accordance with an aspect of the present disclosure.
Figure 4 shows a cross-sectional view of a device for dispensing and applying lotion with the head rotated by 90° in respect of the body.
Figure 5 illustrates the head of an alternative embodiment of a device for dispensing and applying lotion. The other elements of the applicator device are not shown in this figure.
Figure 6 illustrates a detail of an embodiment of the device which includes a pressure sensor and an indicator.
Figure 7 illustrates an exploded detail of an embodiment of the device.
Figure 8 illustrates the cap a) in a view showing the inside including sealing spikes, b) in a view showing the outer surface of the cap.

### Detailed Description of an Example Embodiment

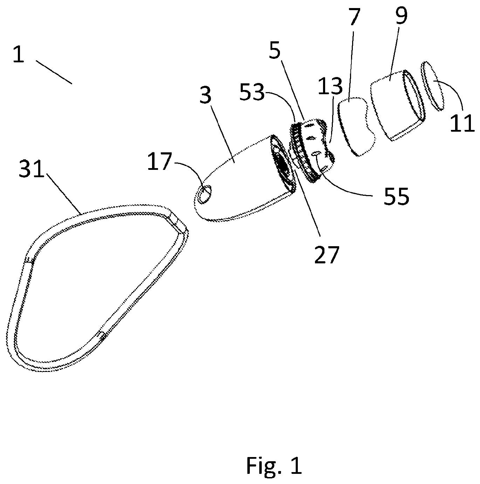

An exploded view of a sun-cream applicator 1 according to the present invention is illustrated in Figure 1. Body 3 which is resiliently deformable connects to head 5 by way of a rotatable mount 27. In the present embodiment, a lotion valve is also present at the interface between body 3 and head 5. The deformable body 3 is hollow and encloses a receptacle 21 for storing sun-cream. The deformable body 3 is made of a material which enables it to be compressed when squeezed by the hand of a user and which tends to return to its uncompressed form once any compression force is released. Examples of suitable materials include polyethylene, polypropylene, polyvinyl chloride, polyethylene terephthalate (PET), high density polyethylene (HDPE) or the like. The head is made from a rigid material which tends to hold its form under pressure, such as a suitable rigid plastic. At least some, if not all of the materials of the applicator device are preferably recyclable.

Head 5 has defined therein a plurality of apertures 57 extending over an area defining a rigid first surface of the head. The apertures fluidically connect to receptacle 21 of body 3, through cavity 29, formed in head 5. That is to say, a fluid connection exists between the receptacle and the one or more apertures. In the present embodiment a lotion valve is present at the fluidic interface between receptacle 21 and cavity 29.

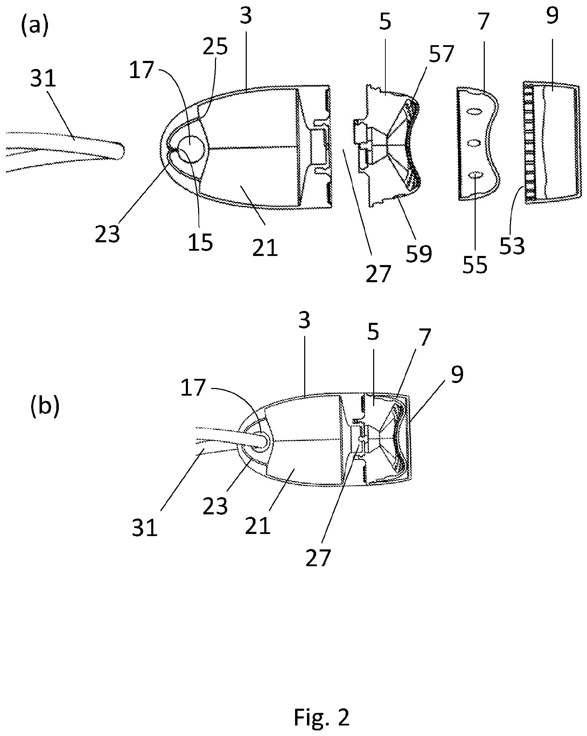

The uppermost surface of rigid head 5 is shaped to facilitate the application of lotion from the absorbent applicator onto a person, in particular onto the face of a person. In the present embodiment the rigid, first surface of the head is saddle shaped. A substantially concave profile is presented in a first direction along the long axis of the elliptical cross-section of the head, and a substantially convex profile is presented in a direction transverse to this. The concave profile along the first direction has a peak at either side. When transferring lotion such as sun-cream from the rigid head of the applicator onto the face of a user, this surface shape has been found beneficial when negotiating facial contours and applying the lotion in an even manner over the face of the user. The rigid first surface comprises a plurality of apertures 57. Capping the head and forming a snug fit thereon is absorbent applicator 7. The absorbent applicator 7 is made from a porous foam material. In the present embodiment protuberances 55 distributed around an outer surface of the head engage with matching indentations present in the under-surface of the absorbent applicator layer (see Figure 2(a)) acting as engagement means to help maintain the snug fit between the rigid head 5 and the absorbent applicator 7.

Cap 9 sits over the absorbent applicator layer 7, protecting the absorbent applicator from the surrounding atmosphere and from soiling. In the present embodiment, cap 9 engages on head 5 via a friction fit 53, enhanced by ridges around the outer circumference of rigid head 5. The cap 9 may be removed when access to the absorbent applicator surface is required, for example when a user wishes to use the device to apply sun-cream to his- or herself, and re-mounted on the rigid head 5 at an appropriate subsequent time. In the embodiment illustrated in Figure 1, the cap also incorporates a mirror 11 or mirrored surface. The user may use this mirror when using the device to apply sun-cream to his or her face, for example, in order to observe the application. The cap 9 may act as a stand, upon which the applicator may be stood.

The body 3 of the applicator has a through-hole 17, passing from one side of the applicator body to the other at the end of the body distant from the head 5. Loop 31 passes through through-hole 17 providing a carrying means by which the device 1 may be carried.

Perpendicular to the main longitudinal axis of the applicator, the applicator of the present embodiment has a generally elliptic cross-section. The outer elliptical shape of the body 3 is the same as the outer elliptical shape of the head 5 where these two elements connect.

Cross-sectional views of the applicator are illustrated in Figure 2(a), which shows an exploded view, and Figure 2(b), which shows an unexploded view. In the present embodiment the receptacle for storing sun-cream 21 is created by having a hollow space within deformable body 3. Air tube 23 connects receptacle 21 to the outside atmosphere. Air inlet entry 15, situated in this embodiment within through-hole 17, provides an entrance for air to enter the receptacle 21. A stop valve is present at air inlet entry 1, enabling air to enter the receptacle but preventing pressurised air within the receptacle from exiting via air tube 23. Air enters through air inlet entry 15, travels through air tube 23 and enters receptacle 21 at air inlet 25. The air enters receptacle 23 at the end distant from head 5.

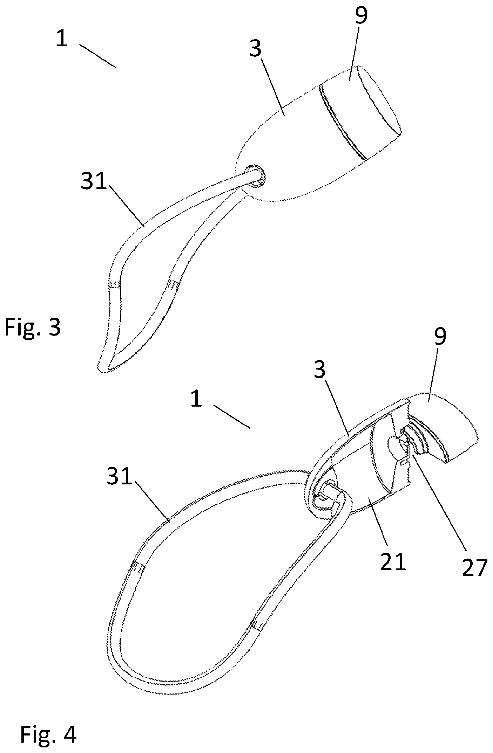

In the unexpanded cross-sectional illustration of Figure 2(b), the orientation of head 5 to body 3 is such that the elliptical cross-sections of these two elements have the same orientation and match up (see also Figure 3). In this orientation of head and body, the lotion valve 27 is closed, preventing free movement of sun-cream between the receptacle 21 and cavity 29 of head 5. This corresponds to the first configuration of the lotion valve.

There is a rotational connection between body 3 and head 5 with an axis of rotation which corresponds to the longitudinal central axis of the body. Head 5 may be rotated relative to body 3 between a first position, in which the elliptically-shaped connecting cross-sections of head 5 and body 3 align (see Figure 2(b) and Figure 3), and a second position, in which the long axis of the elliptically-shaped connecting cross-sections of head 5 is at right angles to the elliptically-shaped connecting cross-sections of body 3 (see Figure 4). In the first position, the lotion valve between receptacle 21 and cavity 29 is closed in a first configuration. In the second position, the lotion valve between receptacle 21 and cavity 29 is open in a second configuration. When the lotion valve between receptacle 21 and cavity 29 is open, sun-cream which is stored in receptacle 21 may be transferred to cavity 29. This may be done for example by squeezing resiliently deformable body 3. From cavity 29, the sun-cream can pass through the apertures 57 in head 5 and into the absorbent applicator foam layer 7. Once the absorbent applicator foam layer is loaded with sun-cream, the user may rotate head 5 to the first position, thereby closing the lotion valve between receptacle 21 and head cavity 29. The user can then rub the absorbent applicator over his or her skin thereby applying sun-cream to the skin, utilising the shape of the applicator head to access the contours of the body or face, and making use of the rigidity provided by the head to help rub the lotion into the skin to maximise protection. In this way protective suncream can be applied without the user having to get any on his or her hands.

In the present embodiment the fluidic connection from receptacle 21 to cavity 29 in head 5 passes via a nominally circular orifice, which is divided into quadrants. Two of the quadrants are open, and two of the quadrants are closed. Similarly, the fluidic connection into cavity 29 of head 5 passes via a nominally circular orifice, which is divided into two open and two closed quadrants. With the head in its first position, its elliptical cross-section aligned up with that of the body (see Figure 3), the closed quadrants of the body close off the fluidic connection which would be given by the open quadrants of the head, and the closed quadrants of the head close off the fluidic connection which would be given by the open quadrants of the body. In this way, the closed quadrants of the head together block off the free fluidic connection between body receptacle 21 and head cavity 29. With the head in its second position, its elliptical cross-section at right angles to that of the body (see Figure 4), the closed quadrants of the body and the closed quadrants of the head overlap, thereby opening a free fluidic connection between body receptacle 21 and head cavity 29 through the open quadrants of body 3 and head 5.

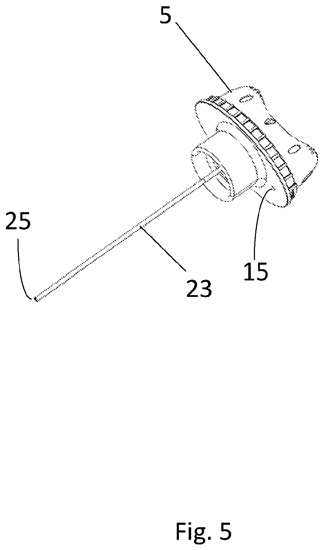

Figure 5 illustrates the head 5 of an alternative embodiment of the applicator. In this embodiment the air inlet entry 15 is located in a surface of the base of head 5. Air tube 23 runs from inlet 15 inside the head 5, passes through the orifice which provides the fluidic connection to receptacle 21, and continues down the length of receptacle 21, along its longitudinal axis, to the end of the receptacle distant from the head. Thus air entering the receptacle 21, for example following deformation of the body by squeezing, is directed to the distal end of the receptacle. As the tube 23 runs down the long central axis of the body, it is unaffected by and also does not affect deformation of the body when it is squeezed. In the embodiment partly illustrated in Figure 5, the head attaches to the body via a screw connection.

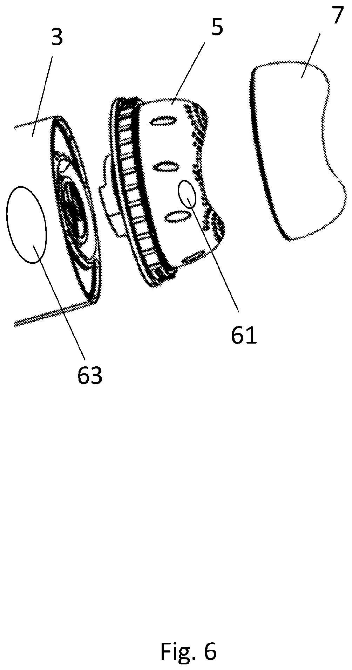

Figure 6 illustrates a detail of an embodiment of the device which includes a pressure sensor 61. Pressure sensor 61 is located on the rigid first surface of head 5 and is sensitive to the application of force to absorbent applicator 7, such as occurs when the device is used to apply sun cream to a user. The body 3 of the applicator houses a lamp and speaker 63 which emit light and sound, respectively, when pressure sensor 61 detects a force. The electrical connection between pressure sensor 61 and lamp and speaker 63 is not illustrated. It will be understood that the device also typically includes a power source, such as a battery (not shown).

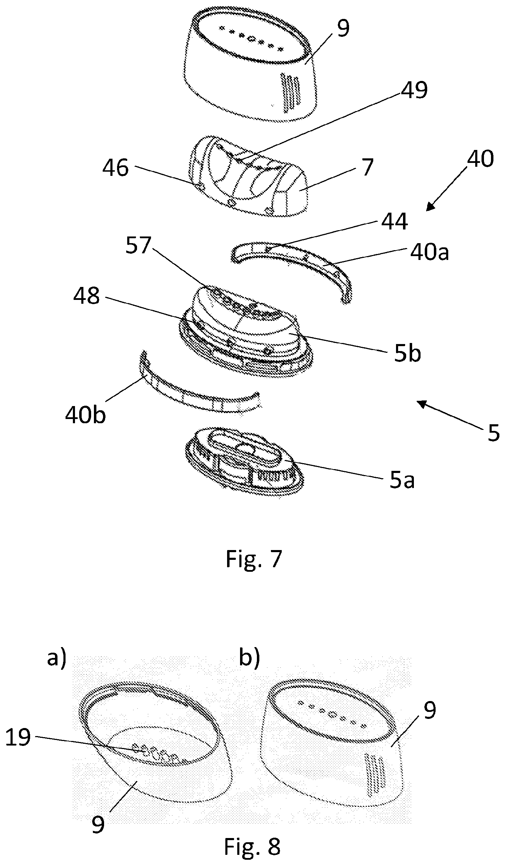

Figure 7 shows an exploded view of a detail of a further embodiment on the present invention.

In this embodiment absorbent applicator 7 comprises a series of holes 46 around the edge of its base. Head 5 in this embodiment comprises two parts: a first head part 5a, which connects to body 3, and a second head part 5b, in which dispensing apertures 57 are present. Second head part 5b additionally comprises six protrusions 48 located around its circumference. Each of these protrusions fits through a corresponding securing hole 46 in the rim of absorbent applicator 7 when the absorbent applicator 7 is mounted on head part 5b. The attachment of absorbent applicator 7 to second head part 5b is made more secure with ring clamp 40, comprised of first ring clamp part 40a and second ring clamp part 40b each of which comprises three pins 44. The pins 44 are arranged to engage with the protrusions 48 located around the circumference of second head part 5b. In this way, absorbent applicator 7 is retained on second head part 5b by protrusions 48 passing through securing holes 46, by the contact on the inner side of the absorbent applicator 7 with outer surface of second head part 5b, and by the contact on the outer side of the absorbent applicator 7 with the main body of ring clamp 40.

Figure 8 shows illustrates the cap 9 of the device according to one embodiment. The cap 9 in this embodiment comprises a series of sealing elements 19 which extend from an internal surface of the cap within the cap. The sealing elements, which are seen in Figure 8(a), are such that when the cap 9 is mounted on the device 1, they engage with corresponding pores in the absorbent applicator 7 and corresponding apertures 49 in head 5. This provides an efficient seal when the cap is mounted, preventing suncream in the device from drying out, even if the device is not used for an extended period of time.

In summary, there is provided a device (1) for dispensing and applying lotion, such as sun-cream. The device comprises a body (3). The body (3) is resiliently deformable and defines a receptacle (21) for storing lotion therein. The device also comprises a head (5) having defined therein one or more apertures (57) extending over an area defining a rigid first surface of the head (5). The body (3) and the head (5) are connected such that a fluid connection exists between the receptacle (21) and the one or more apertures (57) of the head (5). The device also comprises an absorbent applicator (7) capping the rigid first surface of said head (5). The absorbent applicator (7) supported by the rigid first surface of the head (5) may be used to apply sun-cream to a user.

Throughout the description and claims of this specification, the words "comprise" and "contain" and variations of them mean "including but not limited to", and they are not intended to and do not exclude other components, integers, or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, or groups described in conjunction with a particular aspect, embodiment, or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification, as long as these fall under the scope of the appended claims.

## Claims

1. A device (1) for dispensing and applying lotion, the device comprising:
a body (3), the body being resiliently deformable and defining a receptacle (21) for storing lotion therein;
a head (5) having defined therein one or more apertures (57) extending over an area defining a rigid first surface of the head, wherein the body (3) and the head (5) are connected such that a fluid connection exists between the receptacle (21) and the one or more apertures (57) of the head; and
an absorbent applicator (7) capping the rigid first surface of said head;
**characterized in that** at least a region of the first surface of the head defines a substantially concave profile in a first direction, and wherein the at least a region of the first surface of the head defines a substantially convex profile in a second direction, transverse to the first direction.

2. The device according to claim 1, wherein the head defines a cavity, the cavity being part of the fluid connection between the receptacle and the one or more apertures.

3. The device according to claim 1 or claim 2, wherein the rigid first surface of the head defines a shape configured to facilitate the application of lotion from the absorbent applicator onto a person, in particular onto the face of a person.

4. The device according to any preceding claim, wherein the one or more apertures (57) is a plurality of apertures.

5. The device according to any preceding claim, further comprising a lotion valve (27) between the body (3) and the head (5), wherein said lotion valve (27) has a first configuration, in which first configuration passage of lotion from the body to the one or more apertures (57) via the fluid connection is restricted or prevented, and a second configuration, in which second configuration lotion may pass from the body to the one or more apertures (57) via the fluid connection.

6. The device according to any preceding claim, wherein the absorbent applicator (7) is formed from a flexible material, wherein optionally the absorbent applicator (7) is porous.

7. The device according to any preceding claim wherein the device comprises engagement means which serves to retain in place the absorbent applicator (7) capping the rigid first surface of said head, wherein optionally the engagement means comprise a clamp (40) with first cooperating means (44), the first cooperating means (44) engaging with second cooperating means (48) of the head.

8. The device according to any preceding claim, additionally comprising an air inlet (25) allowing air to enter the receptacle, wherein optionally the device is arranged such that air enters the receptacle when the body (3), having been resiliently deformed so as to compress the receptacle (21), is permitted to return to an original shape increasing the volume of the receptacle.

9. The device according to claim 8, wherein the air inlet (25) is arranged to cause air to be directed to a distal end of the receptacle, away from a proximal end of the receptacle, the proximal end being closer to the head than the distal end.

10. The device according to any preceding claim, additionally comprising carrying means (31) with which the device may be carried, optionally wherein the carrying means is a handle or a strap.

11. The device according to any preceding claim, additionally comprising a cap (9) configured to be removably retained on the head (5), wherein optionally the cap (9) provides a stand on which the device may be stood.

12. The device according to claim 11, wherein the cap comprises a sealing element (19) for sealing the receptacle when the cap is retained on the head.

13. The device according to any preceding claim, wherein the device comprises a mirrored surface (11).

14. The device according to any preceding claim, wherein the device comprises a switch, such as a pressure sensor.

15. The device according to claim 14, whereby the switch is functionally connected to an indicator, and wherein the device is configured such that activation of the switch causes operation of the indicator.

## Patentansprüche

1. Vorrichtung (1) zum Abgeben und Auftragen von Lotion, wobei die Vorrichtung umfasst:
einen Körper (3), wobei der Körper elastisch verformbar ist und einen Behälter (21) zum Aufbewahren von Lotion darin definiert;
einen Kopf (5), aufweisend eine oder mehrere darin definierten Öffnungen (57), die sich über einen Bereich erstrecken, der eine starre erste Oberfläche des Kopfes definiert, wobei der Körper (3) und der Kopf (5) derart verbunden sind, dass eine Fluidverbindung zwischen dem Behälter (21) und der einen oder den mehreren Öffnungen (57) des Kopfes besteht; und
einen saugfähigen Applikator (7), der die starre erste Oberfläche des Kopfes abdeckt;
**dadurch gekennzeichnet, dass** mindestens ein Bereich der ersten Oberfläche des Kopfes ein im Wesentlichen konkaves Profil in einer ersten Richtung definiert, und wobei der mindestens eine Bereich der ersten Oberfläche des Kopfes ein im Wesentlichen konvexes Profil in einer zweiten Richtung, quer zur ersten Richtung, definiert.

2. Vorrichtung nach Anspruch 1, wobei der Kopf einen Hohlraum definiert, wobei der Hohlraum Teil der Fluidverbindung zwischen dem Behälter und der einen oder den mehreren Öffnungen ist.

3. Vorrichtung nach Anspruch 1 oder Anspruch 2, wobei die starre erste Oberfläche des Kopfes eine Form definiert, die konfiguriert ist, um das Auftragen von Lotion aus dem saugfähigen Applikator auf eine Person, insbesondere auf das Gesicht einer Person, zu erleichtern.

4. Vorrichtung nach einem der vorstehenden Ansprüche, wobei die eine oder mehreren Öffnungen (57) eine Vielzahl von Öffnungen sind.

5. Vorrichtung nach einem der vorstehenden Ansprüche, ferner umfassend ein Lotionsventil (27) zwischen dem Körper (3) und dem Kopf (5), wobei das Lotionsventil (27) eine erste Konfiguration aufweist, in welcher ersten Konfiguration der Durchgang von Lotion von dem Körper zu der einen oder den mehreren Öffnungen (57) über die Fluidverbindung eingeschränkt oder verhindert ist, und eine zweite Konfiguration, in welcher zweiten Konfiguration Lotion von dem Körper zu der einen oder den mehreren Öffnungen (57) über die Fluidverbindung gelangen kann.

6. Vorrichtung nach einem der vorstehenden Ansprüche, wobei der absorbierende Applikator (7) aus einem flexiblen Material ausgebildet ist, wobei der absorbierende Applikator (7) optional porös ist.

7. Vorrichtung nach einem der vorstehenden Ansprüche, wobei die Vorrichtung Eingriffsmittel umfasst, die dazu dienen, den saugfähigen Applikator (7), der die starre erste Oberfläche des Kopfes abdeckt, an Ort und Stelle zu halten, wobei optional die Eingriffsmittel einen Bügel (40) mit ersten zusammenwirkenden Mitteln (44) umfassen, wobei die ersten zusammenwirkenden Mittel (44) mit zweiten zusammenwirkenden Mitteln (48) des Kopfes in Eingriff stehen.

8. Vorrichtung nach einem der vorstehenden Ansprüche, zusätzlich umfassend einen Lufteinlass (25), der es Luft ermöglicht, in den Behälter einzutreten, wobei die Vorrichtung optional derart eingerichtet ist, dass Luft in den Behälter eintritt, wenn es dem Körper (3), nachdem er elastisch verformt wurde, um den Behälter (21) zusammenzudrücken, gestattet wird, in eine ursprüngliche Form zurückzukehren, wodurch das Volumen des Behälters vergrößert wird.

9. Vorrichtung nach Anspruch 8, wobei der Lufteinlass (25) so angeordnet ist, dass er bewirkt, dass Luft auf ein distales Ende des Behälters gerichtet wird, weg von einem proximalen Ende des Behälters, wobei das proximale Ende näher an dem Kopf liegt als das distale Ende.

10. Vorrichtung nach einem der vorstehenden Ansprüche, zusätzlich umfassend ein Tragemittel (31), mit dem die Vorrichtung getragen werden kann, optional wobei das Tragemittel ein Griff oder ein Gurt ist.

11. Vorrichtung nach einem der vorstehenden Ansprüche, zusätzlich umfassend eine Kappe (9), die konfiguriert ist, um abnehmbar an dem Kopf (5) gehalten zu werden, wobei optional die Kappe (9) einen Ständer bereitstellt, auf dem die Vorrichtung abgestellt werden kann.

12. Vorrichtung nach Anspruch 11, wobei die Kappe ein Dichtelement (19) zum Abdichten des Behälters umfasst, wenn die Kappe auf dem Kopf gehalten wird.

13. Vorrichtung nach einem der vorstehenden Ansprüche, wobei die Vorrichtung eine verspiegelte Oberfläche (11) umfasst.

14. Vorrichtung nach einem der vorstehenden Ansprüche, wobei die Vorrichtung einen Schalter, wie einen Drucksensor, umfasst.

15. Vorrichtung nach Anspruch 14, wobei der Schalter funktionell mit einer Anzeige verbunden ist, und wobei die Vorrichtung so konfiguriert ist, dass eine Aktivierung des Schalters einen Betrieb der Anzeige bewirkt.

## Revendications

1. Dispositif (1) permettant de distribuer et d'appliquer une lotion, le dispositif comprenant :
un corps (3), le corps étant élastiquement déformable et définissant un réceptacle (21) pour stocker une lotion dans celui-ci ;
une tête (5) ayant, définies dans celle-ci, une ou plusieurs ouvertures (57) s'étendant par-dessus une zone définissant une première surface rigide de la tête, dans lequel le corps (3) et la tête (5) sont raccordés de telle sorte qu'un raccordement fluidique existe entre le réceptacle (21) et la ou les ouvertures (57) de la tête ; et
un applicateur absorbant (7) coiffant la première surface rigide de ladite tête ;
**caractérisé en ce qu'**au moins une région de la première surface de la tête définit un profil sensiblement concave dans une première direction, et dans lequel l'au moins une région de la première surface de la tête définit un profil sensiblement convexe dans une seconde direction, transversale à la première direction.

2. Dispositif selon la revendication 1, dans lequel la tête définit une cavité, la cavité faisant partie du raccordement fluidique entre le réceptacle et la ou les ouvertures.

3. Dispositif selon la revendication 1 ou la revendication 2, dans lequel la première surface rigide de la tête définit une forme conçue pour faciliter l'application de lotion à partir de l'applicateur absorbant sur une personne, en particulier sur le visage d'une personne.

4. Dispositif selon l'une quelconque des revendications précédentes, dans lequel la ou les ouvertures (57) sont une pluralité d'ouvertures.

5. Dispositif selon l'une quelconque des revendications précédentes, comprenant en outre une valve à lotion (27) entre le corps (3) et la tête (5), dans lequel ladite valve à lotion (27) a une première configuration, première configuration dans laquelle un passage de lotion du corps vers la ou les ouvertures (57) par l'intermédiaire du raccordement fluidique est restreint ou empêché, et une seconde configuration, seconde configuration dans laquelle une lotion peut passer du corps vers la ou les ouvertures (57) par l'intermédiaire du raccordement fluidique.

6. Dispositif selon l'une quelconque des revendications précédentes, dans lequel l'applicateur absorbant (7) est formé à partir d'un matériau flexible, dans lequel, facultativement, l'applicateur absorbant (7) est poreux.

7. Dispositif selon l'une quelconque des revendications précédentes, dans lequel le dispositif comprend un moyen de mise en prise qui sert à retenir en place l'applicateur absorbant (7) coiffant la première surface rigide de ladite tête, dans lequel, facultativement, les moyens de mise en prise comprennent un dispositif de serrage (40) avec des premiers moyens de coopération (44), les premiers moyens de coopération (44) venant en prise avec des seconds moyens de coopération (48) de la tête.

8. Dispositif selon l'une quelconque des revendications précédentes, comprenant en outre une entrée d'air (25) permettant à de l'air d'entrer dans le réceptacle, dans lequel, facultativement, le dispositif est agencé de telle sorte que de l'air entre dans le réceptacle lorsque le corps (3), ayant été déformé par élasticité de façon à comprimer le réceptacle (21), est autorisé à reprendre une forme initiale augmentant le volume du réceptacle.

9. Dispositif selon la revendication 8, dans lequel l'entrée d'air (25) est agencée pour amener de l'air à être dirigé vers une extrémité distale du réceptacle, à l'écart d'une extrémité proximale du réceptacle, l'extrémité proximale étant plus proche de la tête que l'extrémité distale.

10. Dispositif selon l'une quelconque des revendications précédentes, comprenant en outre un moyen de transport (31) avec lequel le dispositif peut être transporté, facultativement dans lequel le moyen de transport est une poignée ou une sangle.

11. Dispositif selon l'une quelconque des revendications précédentes, comprenant en outre une coiffe (9) conçue pour être retenue de manière amovible sur la tête (5), dans lequel, facultativement, la coiffe (9) fournit un support sur lequel le dispositif peut être posé.

12. Dispositif selon la revendication 11, dans lequel la coiffe comprend un élément d'étanchéité (19) pour assurer l'étanchéité du réceptacle lorsque la coiffe est retenue sur la tête.

13. Dispositif selon l'une quelconque des revendications précédentes, dans lequel le dispositif comprend une surface miroir (11).

14. Dispositif selon l'une quelconque des revendications précédentes, dans lequel le dispositif comprend un commutateur, tel qu'un capteur de pression.

15. Dispositif selon la revendication 14, moyennant quoi le commutateur est connecté fonctionnellement à un indicateur, et dans lequel le dispositif est configuré de telle sorte qu'une activation du commutateur provoque le fonctionnement de l'indicateur.
